# EUROPEAN PATENT SPECIFICATION

(11) **EP 1 996 089 B1**
(45) Date of publication and mention of the grant of the patent: **24.08.2016**
(21) Application number: 07753512.8
(22) Date of filing: 20.03.2007
(51) Int. Cl.: A61B 17/22

(54) **MEDICAL GRASPING DEVICE**
MEDIZINISCHES GREIFINSTRUMENT
DISPOSITIF MÉDICAL D'AGRIPPEMENT

(30) Priority: 20.03.2006 US 784126 P
(43) Date of publication of application: 03.12.2008
(73) Proprietor: William A. Cook Australia Pty. Ltd., Brisbane, Queensland 4113 (AU); Cook Medical Technologies LLC, Bloomington, IN 47404 (US)
(72) Inventor: HARTLEY, David, Ernest, Subiaco, Western Australia 6008 (AU); IVANCEV, Krasnodar, S-SE-222 21 Lund (SE); DUCKE, Werner, D., Greenwood, Western Australia 6024 (AU); DEBRUYNE, Michael, P., Bloomington, IN 47408 (US); DIAMOND, Jarett, Bloomington, IN 47408 (US); FRYE, Mark, R., Bloomington, IN 47408 (US)
(74) Representative: Ellis, Katherine Elizabeth Sleigh
(86) International application number: PCT/US2007/006890
(87) International publication number: WO 2007/109257

(56) References cited:
- US-A- 5 098 440
- US-A1- 2002 107 526
- US-A1- 2003 225 419
- US-A1- 2005 085 846

## Description

### Technical Field

This invention is related to medical devices and in particular to a medical grasping device.

### Background of the Invention

There is a current trend in medicine to minimize surgical and interventional procedures, concomitant with the development of minimally invasive tools to access, visualize, infuse, treat, medicate, sample, and interact with internal structures of the body. Occasionally, devices such as catheters, balloons or wires are inadvertently severed in a blood vessel, cavity or organ. Depending on its location, the severed device or fragment must be retrieved. Frequently, a surgical approach is dangerous and costly. In many cases, access has already been established to the severed device, fragment, or foreign body in question, and it is just a matter of locating and removing the foreign body without doing harm to surrounding tissue or forcing it further out of reach.

Certain medical devices are known that are utilized in the ducts and vessels of a human or veterinary patient for retrieval of bodies from the patient. For example, retrieval devices are known for removing calculi such as kidney stones or gallstones from a patient, where the retrieval device is delivered to the target site via the urethra or biliary duct, respectively. The device's distal tip is adapted to deploy at the site to form a basket shape to trap the calculi after which the basket is collapsed to grasp the calculi. Both the device and the grasped calculi are then withdrawn from the patient.

One such stone retrieval device is disclosed in U.S. Pat. No. 5,989,266, in which several loops of wire are caused to emerge from the distal end of a sheath that has previously been delivered through the renal or biliary system of a patient to the site of the stone. The stone becomes trapped within the loops, after which the loops are pulled proximally mostly into the sheath, grasping the stone firmly, after which the sheath, loops and stone are withdrawn from the patient. The loops are disclosed to be made from a superelastic alloy such as Nitinol to automatically form the loops when caused to emerge from the sheath's distal tip. Other similar stone retrieval devices are disclosed in U.S. Pat. Nos. 5,057,114; 5,064,428; 5,133,733 and 5,484,384.

However, use of such devices is not satisfactory for grasping such an object within the vascular system of a patient for repositioning of that object, or for removal of objects from within the vascular system of a patient. For example, in certain situations it is desired to reposition a stent or stent graft within the vasculature, or to retrieve or reposition a badly positioned or misplaced embolization coil. Moreover, during delivery and deployment of a bifurcated stent graft at the site of an abdominal aortic aneurysm, when surgical access has been obtained through the femoral arteries on both sides of the groin, it is desirable to grasp the distal tip of a guide wire extending into the aneurysm from the contralateral iliac artery, to be pulled into the ipsilateral iliac artery at the vessel's aorto-iliac bifurcation, for eventual placement of the contralateral leg extension of the stent graft.

For vascular use, another known device is a suture loop on a catheter distal tip. Yet another is a guide wire that has been doubled over and extended through a catheter so that its distal end forms into a loop that extends axially from the catheter's distal end to be utilized as a retriever when it is pulled proximally to capture an object and hold it against the catheter distal end for withdrawal, sold as the Curry Intravascular Retriever Set by Cook, Incorporated, Bloomington, Indiana, USA. A version of the stone basket device, having helical loops, has been utilized for intravascular retrieval, the Dotter Intravascular Retriever Set also sold by Cook, Incorporated.

In U.S. Pat. No. 5,171,233 is disclosed a snare-type probe for intravascular use. After a catheter is inserted into the patient's vascular system to the site of the foreign object, an elongate member having a loop-shaped distal segment is inserted into the proximal end of the catheter's lumen until the loop-shaped distal segment emerges from the catheter's distal tip at the site. Then the loop-shaped segment extends at an angle to the adjacent portion of the member and opens into a loop. Once a free end of the foreign object is snared within the loop-shaped distal segment as determined by fluoroscopic equipment, the loop-shaped distal segment is pulled proximally into the catheter distal end, collapsing about the ensnared foreign body fragment and holding the foreign body at the distal tip of the catheter during withdrawal. The elongate member is preferably disposed within an outer sheath and is disclosed to be one wire, or two gripped-together wires, of a shape memory material such as a superelastic Nitinol alloy, with a single preformed loop shape at the distal segment defined by two wire portions. The use of Nitinol enables the wire segments defining the distal segment to be straightened and collapsed upon one another into an elastically deformed configuration to pass through the lumen of the catheter and yet automatically open into a loop and extend at a substantial angle upon emerging from the catheter distal tip. One characteristic of this design is that during retraction after grasping, the loop quickly changes, or 'flips' between the angled orientation and a generally axial one, and this results in less assured control over the item during grasping, and commonly will result in escape of the item thus requiring redeployment of the loop for another grasping attempt.

US 2002/0107526, which discloses the features defined in the preamble of independent claim 1, describes a medical grasping device for vascular use, having an outer sheath, an elongate control member extending within an outer sheath to a distal tip section, and a proximal control assembly including an actuation section joined to the elongate control member. Adjacent to the distal tip section is a grasping portion that is extendable from the outer sheath to create loops for grasping a target object (T) for repositioning within the vascular system, or for removal from the patient, with loops being retractable into the outer sheath to hold the target object against the device during movement of the device. Elongate control member is preferably a cannula or tube having a lumen extending completely therethrough for placement over a guide wire already in the patient.

### Summary of the Invention

The present invention seeks to provide a grasping device for grasping and for removing or repositioning an object within the vascular system of a patient, such as a stent, stent graft, embolization coil, filter, occlusion device, as well as a distal tip of a catheter or a guide wire, pacemaker lead or any such item or item part located within a patient.

According to an aspect of the present invention, there is provided a medical grasping device as specified in claim 1.

Advantageously, said elongate control member is a flexible cannula defining a lumen extending therethrough into which a guide wire is receivable and movable with respect thereto.

Preferably said outer sheath is flexible and kink-resistant and has lubricious outer and inner surfaces.

Preferably said control assembly includes an actuation section that is easily able to be gripped for reciprocal movement along a handle to actuate said elongate control member with respect to said outer sheath to deploy and retract said grasping member, respectively. The actuation section may include a connecting block affixed to said elongate control member and is disposed within a longitudinal slot in said handle and is movable along said slot between opposite ends thereof.

The control assembly can further include a pin vice assembly at the proximal end thereof, the pin vice assembly acting between the control member and the control assembly to prevent relative movement therebetween when engaged.

Preferably the control member comprises an outer rigid tube and an inner flexible tube, the rigid tube extending through the control assembly and into the outer sheath and moveable therethrough. The connecting block can be affixed to the outer rigid tube.

Preferably each of said wire loops includes an arcuate outer section having a radius about equal to a radius of a deployment site of a vessel into which the grasping device is inserted.

Preferably said grasping member comprises a plurality of wire loops that each are formed from a superelastic alloy.

Preferably said grasping member comprises a plurality of wire loops having proximal end portions that are joined to said elongate control member at affixation joints and initially extend axially from said elongate control member even when said wire loops emerge from said distal end of said outer sheath and self-deploy transversely of a longitudinal axis of the grasping device. The affixation joints can comprise a spiral of the wire of the wire loops around the elongate control member.

Preferably said wire loops comprise Nitinol wire segments, the distal tip comprises an atraumatic section, and/or the elongate control member is formed for low elongation.

Preferably each of said wire loops includes an arcuate outer section which comprises or includes a radio opaque material. The radio opaque material can comprise a coil of platinum wire around and extending along the arcuate outer section.

The device can include a port fitting to allow flushing with sterile saline solution between the elongate control member and the outer sheath to eliminate air, while the device is outside of the patient. An air seal can be utilized near the distal end of the sheath. Preferably each of said wire loops includes side sections that overlap substantially completely with side sections of adjacent ones of said wire loops on each side thereof.

The medical grasping device may comprise: an elongate control member provided with an atraumatic distal tip section and a proximal end portion, said elongate control member including a grasping member proximal said distal tip section, an outer sheath with a passageway therethrough surrounding said elongate control member and being movable with respect thereto; and a control assembly disposed at a proximal end of said outer sheath and said proximal end portion of said elongate control member and in operative relation thereto for urging said grasping member from a distal end of said outer sheath and retraction thereinto, said grasping member comprising a plurality of pre-formed wire loops which self-deploy transversely upon emerging from said distal end of said outer sheath, respective ends of each wire loop being fastened to substantially opposite sides of the elongate control member whereby each of said wire loops is substantially semi-circular upon full deployment and the respective ends of each wire loop extend substantially in opposite directions from the elongate control member, wherein each of said wire loops includes side sections that overlap substantially completely with side sections of adjacent ones of said wire loops on each side thereof, said elongate control member is a flexible cannula providing a lumen extending therethrough into which a guide wire is receivable and movable with
respect thereto, said control assembly including an actuation section that is grippable for reciprocal movement along a handle to actuate said elongate control member with respect to said outer sheath to deploy and retract said grasping member, respectively, and said actuation section includes a connecting block affixed to said elongate control member and is disposed within a longitudinal slot of said handle and is movable along said slot between opposite ends thereof and said connecting block comprises a rigid tube through which extends the elongate control member and the rigid tube extending into the outer sheath and moveable therethrough, the grasping member comprising four preformed wire loops from a superelastic alloy that self-deploy transversely upon emerging from said distal end of said outer sheath approximately equally spaced angularly about a longitudinal axis of said elongate control member and thereby generally occupy a full cross-section of a vessel into which the grasping device is inserted, the wire loops including proximal end portions that are joined to said elongate control member at affixation joints and the affixation joints comprise a spiral of the wire of the wire loops around the elongate control member.

The preferred embodiment can provide a low profile, medical grasping device that is conformable to the vascular anatomy while generating a substantial tensile force.

It is further desired to provide such a device that is trackable through the vascular system over a guide wire already in situ.

Preferably, the device is atraumatic to the patient.

An air seal can be utilized near the distal end of the sheath.

The grasping device may include a proximal control assembly that is easily manipulated for actuation during grasping, and for assured continued automatic grasping of the object with a controlled, limited amount of force while the device is being moved to manually reposition the object or to remove it completely. The elongate control member is preferably formed to have torqueability and significant tensile strength with low elongation distal to the proximal end portion. The outer sheath can have a flexible but kink-resistant construction with lubricious outer and inner surfaces.

### Brief Descriotion of the Drawing

Embodiments of the present invention are described below, by way of example only, with reference to the accompanying drawings, in which:
Figure 1 shows a first embodiment of medical grasper according to the present invention;
Figures 2 the operation of the medical grasper shown in Figure 1;
Figure 3 shows a longitudinal cross sectional view of the medical grasper shown in Figure 1;
Figures 3A to 3D show cross sectional details of various portions of the device shown in Figure 3;
Figure 4 shows a longitudinal cross sectional view of the medical grasper shown in Figure 2;
Figures 5A to 5C shows various stages of the deployment of the grasping members in end view;
Figures 6A to 6C shows various stages of the deployment of the grasping members in side view;
Figure 7 shows a schematic view of the grasping member expanded into a vessel.
Figure 8 shows the detail of one embodiment of the grasping member and fastening of the grasping member onto the control member;
Figure 9 shows another embodiment of grasping member particularly including radio opaque marking on the wire loops; and
Figure 10 show a detail of the radio opaque marking onto a wire loop.

### Detailed Description

Throughout this specification the term distal with respect to a portion of the medical grasping device means the end of the medical grasping device further away from the user and the term proximal means the portion of the medical grasping device closer to the user.

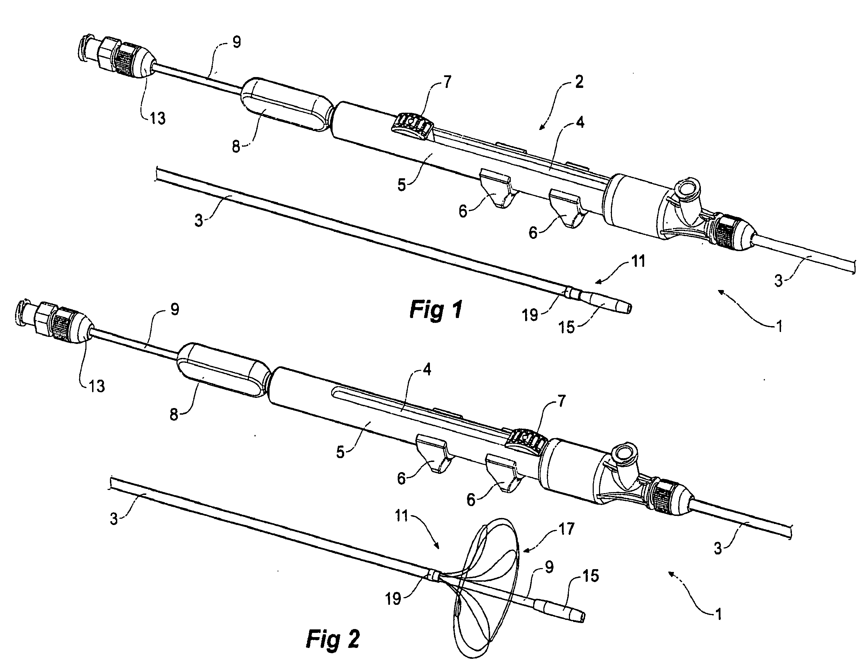

Now looking more closely at the drawings and first in relation to the embodiment shown in Figures 1 to 4, it will be seen that the medical grasper 1 includes a control assembly 2 and a sheath 3 extending from the control assembly. The control assembly 2 comprises a fixed handle 5 and a sliding handle 7. The control assembly 2 includes gripper protrusions 6 on the fixed handle 5 to enable a physician to grip the fixed handle.

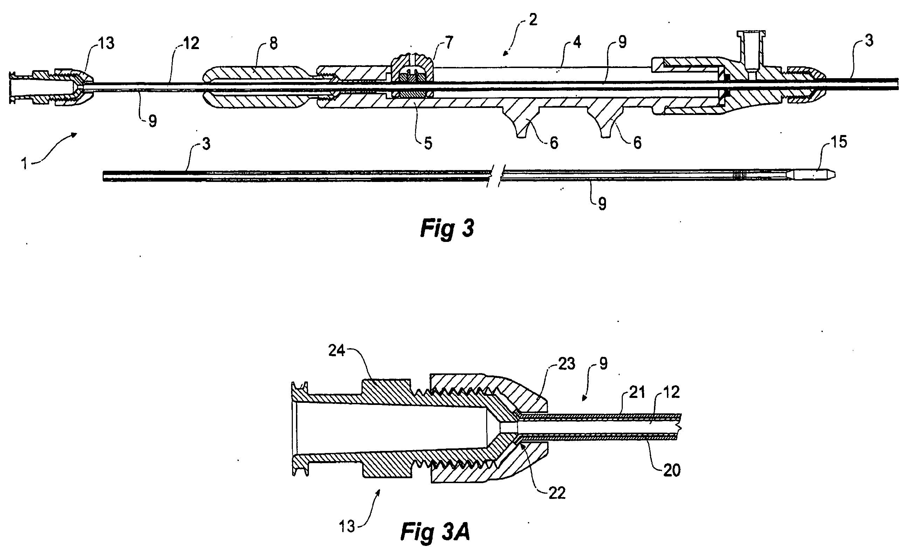

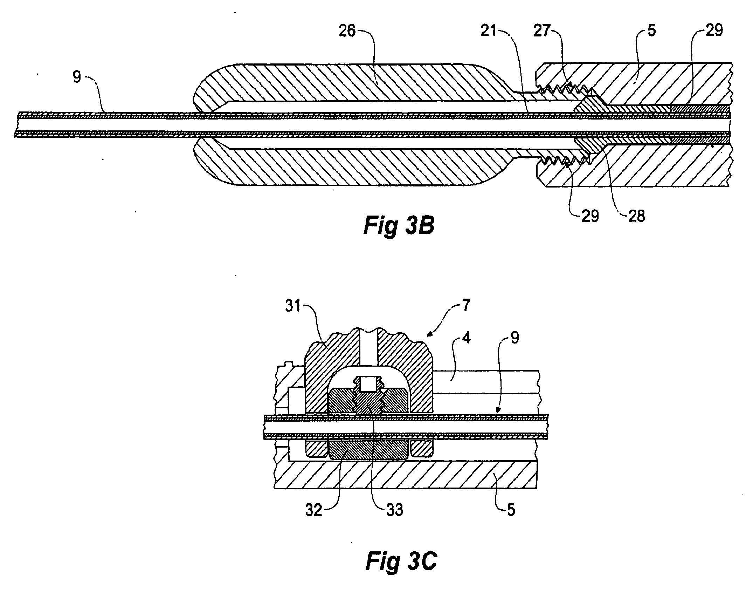

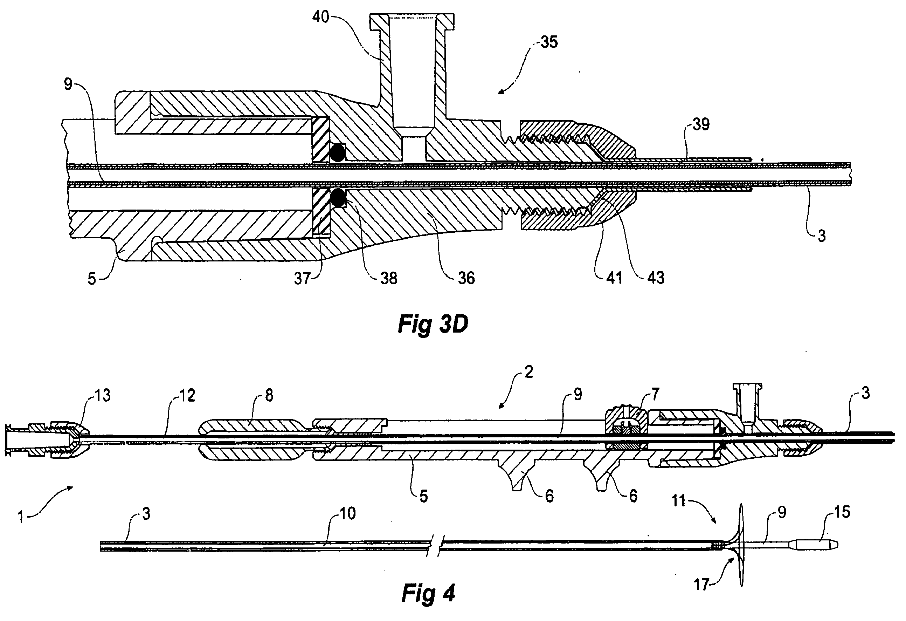

A control member 9, which can be particularly seen in Figures 3 and 4, extends from a distal end 11 of the medical grasping device 1 through the sheath 3 and is affixed to the sliding handle 7 in the control assembly 2. The control member 9 then extends further through the control assembly 2 via a locking pin vice 8 to a Luer lock connector 13 at the proximal end of the medical grasping device. The pin vice enables the control member to be locked in a set position. This is particularly useful during advancement and retraction of the medical grasper through the vasculature.

The distal end of the control member 9 in this embodiment terminates in an atraumatic tip 15. The control member 9 comprises a cannula 10 with a longitudinal lumen 12 therethrough through which a guide wire can be passed. The medical grasping device can be deployed over a guide wire by extending the guide wire through the lumen 12 of the control member 9. At the distal end of the sheath 3 and there is a radio opaque marker 19 on the control member 9 just proximal of the atraumatic tip 15. The atraumatic tip may be formed from soft nylon or radio opaque urethane material.

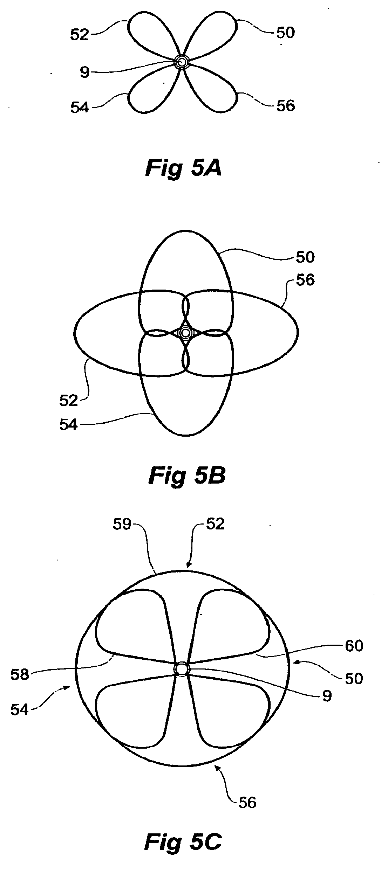

As can be seen in Figures 2 and 4, the sliding handle 7 has been moved towards the distal end of the control assembly 2 along elongate slot 4 in the fixed handle 5 and this has extended the control member 9 distally to expose a grasping member 17. In Figure 1 the grasping member is trapped between the sheath 3 and the control member 9 but when the control member is extended distally, the grasping member 17 can expand out. The grasping member comprises a plurality of wires of a shape memory material, which are formed into the extended shape as shown in Figure 2 and then withdrawn into the sheath as shown in Figure 1 for deployment. More detail of the grasping member is shown in Figures 5 to 8.

The medical grasping device disclosed herein is used to grasp objects and particularly guide wires or the like within a lumen of the human or animal body. The medical grasping device is deployed over a guide wire into a lumen of the body in the form shown in Figure 1 and then the grasping member 17 is extended as shown in Figure 2. The article such as a guide wire to be grasped is then deployed so that the guide wire extends through the loops of the grasping member and then the sliding handle 7 is retracted to retract the grasping member loops into the sheath until the guide wire has been trapped by the loops and grasped by the engagement of the sheath 3 with the loops of the grasping member 17, and then the guide wire can be withdrawn from the lumen by withdrawal of the whole device, while keeping tension on the sliding handle 7 to prevent release of the guide wire 19. Advantageously the pin vice 8 is tightened before retraction to assist with maintaining grip on the grasped object.

The control member 9 is a composite construction consisting of a flexible tube surrounded by a more rigid tube as shown in detail in Figure 3A. At its proximal end the more rigid tube of the control member 9 extends from the Luer lock connector 13 at the proximal end of the medical grasping device through the control assembly 2 and partially into the sheath 3. The flexible tube can be constructed from a 4.1 French braided nylon catheter and the more rigid tube can be a stainless steel catheter. Both catheters are flared at their proximal ends and clamped into the Luer lock connector 13. The sliding handle or knob clamps onto the more rigid tube as shown in detail in Figure 3C.

Figures 3A to 3D show detail of various portions of the medical grasper and particularly parts of the control assembly.

Figure 3A shows the Luer lock connector 13 and its connection with the control member 9. The control member 9 comprises a flexible tube 20 surrounded by a more rigid tube 21. The flexible tube 20 has the longitudinal lumen 12 therethrough through which a guide wire can be passed. At their proximal ends 22 they are both flared and clamped between a nut 23 and a Luer lock connector body 24.

Figure 3B shows the pin vice assembly 8. The pin vice assembly comprises a knob 26 with a screw thread 27 which engages with a screw thread 29 in the rear of the fixed handle 5 and by rotation of the knob 26 causes the knob to engage against a clamp member 28 and this in turn causes the clamp member to clamp against the outer rigid tube 21 of the control member 9. This prevents movement of the control member with respect to the fixed handle 5.

Figure 3C shows detail of the sliding handle 7 and its connection with the control member 9. The sliding handle 7 has a gripping knob 31 which engages around a clamping block 32. The clamping block 32 has a set screw 33 which engages against the outer rigid tube 21 of the control member 9. Movement of the sliding handle 7 along the slot 4 in the fixed handle 5 will cause the control member to move also provided the pin vice 8 has been disengaged.

Figure 3D shows a haemostatic seal arrangement between the control member 9 and the fixed handle 5 and the connection of the sheath 3 with the fixed handle 5. The distal end 35 of the fixed handle 5 comprises an adaptor 36 with a combination of a silicone washer seal 37 and an O-ring seal 38 engaged between the adaptor 36 and fixed handle 5. This provides a haemostatic seal for the lumen 39 between the sheath 3 and the control member 9. A flushing port 40 enables supply of flushing fluid into the lumen 39 between the sheath 3 and the control member 9 to allow for instance flushing with sterile saline solution between the elongate control member and the outer sheath to eliminate air, while the device is outside of the patient. The sheath 3 is clamped onto the adaptor 36 by a clamping nut 41 which engages against a flare 43 at the proximal end of the sheath 3.

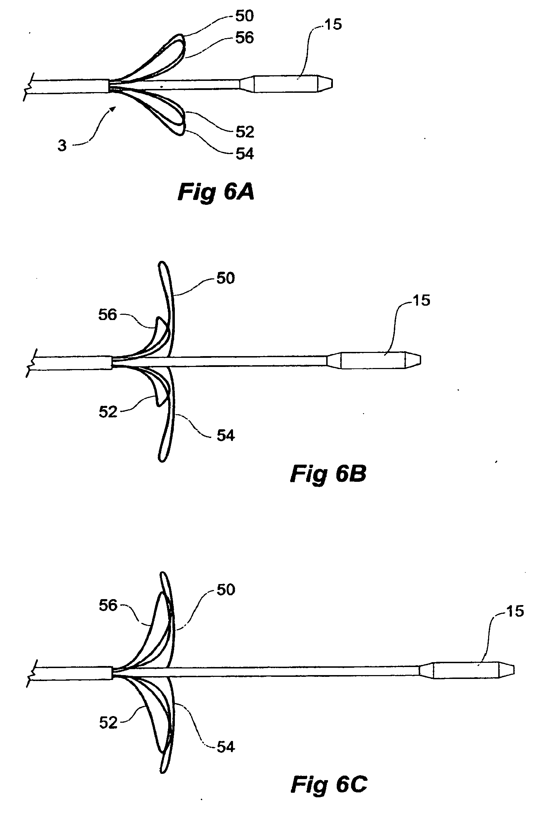

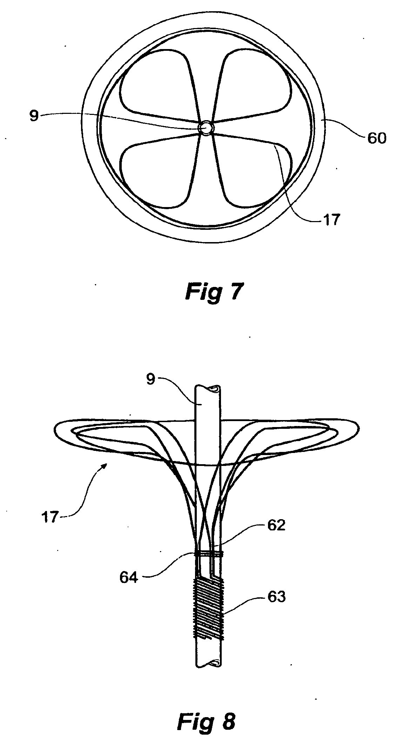

Figures 5A to 5C and 6A to 6C show various stages of the deployment of the grasping member 17 from the end of the sheath 3. The grasping member 17 comprises a number of loops of a shape memory wire, such as Nitinol™ wire, which are fastened to the control member 9. One method of fastening the wires to the control member is discussed in Figure 8 below. In this embodiment there are four loops of wire 50, 52, 54 and 56 extending from the control member 9. Each wire loop extends substantially radially outwards from the control member in a first portion 58 and then substantially circumferentially in an arcuate portion 59 before extending radially inwards a portion 60 to the approximate diametrically opposite part of the control member 9, as can be best seen in Figure 5C. By this arrangement each loop 50, 52, 54 and 56 covers approximately an area of half of a circle and each wire loop overlaps its neighbour on either side by approximately one quarter of a total circle area.

Figures 5A and 6A show an initial stage of deployment where the loops of wire 50, 52, 54 and 56 are only just starting to emerge from the sheath from where they are nestled behind the atraumatic tip 15.

Figures 5B and 6B show a further stage of deployment where the loops of wire 50, 52, 54 and 56 are spreading under the influence of their shape memory and are beginning to overlap.

Figures 5C and 6C show the final stage of deployment where the loops of wire 50, 52, 54 and 56 are fully deployed and each wire loop overlaps its neighbours on either side by approximately one quarter of a total circle area.

By this arrangement, as shown in Figure 7 where the grasping member 17 has been deployed into a vessel 60, the wire loops of the grasping member extend right out to the walls of the vessel and completely encircle the walls so any device to be gripped which is passed down through the vessel will be encompassed and conveniently gripped.

It will be realised that the deployed diameter of the grasping member should be selected for the expected diameter of the vessel into which the grasping member is to be used.

One method of affixing the wire loops of the grasping member to the control member according to one embodiment of the present invention is shown in Figure 8.

As discussed above, the wire loops of the grasping member 17 are formed from a shape memory material such as Nitinol™. Before being placed onto the control member 9, the wire loops are formed on a mandrel with adjacent wires of adjacent loops 62 wound in a series of spirals 63 around a mandrel and then suitably heat treated. After heat treatment, the mandrel is removed and the control member 9 is deployed through the spiral of wire loops so that the spiral of wire loops grips the control member. A binding 64 is placed around the pairs of adjacent wires to keep them adjacent to the control member 9 and a suitable sealing material can be placed over the spiral 63.

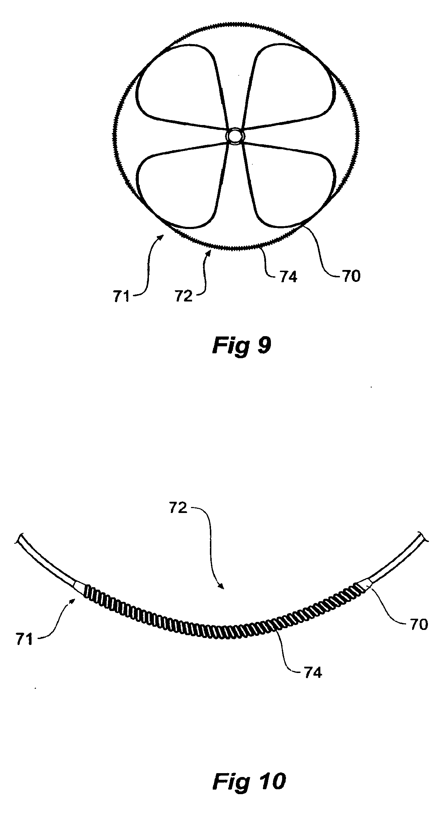

Figure 9 shows an end on view of another embodiment of grasping member 71 according to this invention. In this embodiment, each of the wire loops 70 of the grasping member 71 in its outer region 72 has formed thereon a coil 74 of fine platinum wire to enhance the radio opaque nature of the wire loops.

Figure 10 shows a detail of the wire loops with the platinum wire coil 74 formed thereon. At each end of the coil 74, a portion of non toxic adhesive 76 is provided to provide a tapered transition from the coil 74 to the wire 72.

The embodiments of medical grasping device disclosed herein can be useful in any multiple access vascular procedure for adjusting the final position of a medical device, such as through the iliac or subclavian arteries. They can additionally be useful with the liver or kidney or any other nonvascular procedure, especially where access to the site involves a tortuous path, since the grasping device is flexible and is adapted to follow a guide wire.

## Claims

1. A medical grasping device (1) including:
an elongate control member (9) provided with a distal tip (15) and a proximal end portion, said elongate control member including a grasping member (17) proximal said distal tip;
an outer sheath (3) with a passageway within which the elongate control member is slidably held;
and a control assembly (2) disposed at a proximal end of said outer sheath and said proximal end portion of said elongate control member and operable to urge said grasping member out from and into a distal end of said outer sheath;
said grasping member (17, 71) comprising four pre-formed wire loops (50, 52, 54, 56, 70) approximately equally spaced angularly about a longitudinal axis of said elongate control member (9), which self-deploy from said distal end of said outer sheath, respective ends of each wire loop being fastened to the elongate control member; wherein said wire loops, when deployed, extend in different directions and wherein adjacent wire loops overlap with one another;
**characterized in that** each of said wire loops is substantially semicircular when fully deployed and each wire loop overlaps its neighbour on either side by approximately one quarter of a total circle area so as to provide a generally round grasping member.

2. A grasping device (1) according claim 1, wherein said elongate control member is a flexible cannula (9) providing a lumen extending therethrough into which a guide wire (19) is slidably receivable.

3. A grasping device (1) according to claim 1 or 2, wherein said outer sheath (3) is flexible and kink-resistant and has a lubricious outer and/or inner surface.

4. A grasping device (1) according to claim 1, 2 or 3, wherein said control assembly includes a grippable actuation element (7) reciprocally movable along a handle (5) to actuate said elongate control member (9) with respect to said outer sheath (3) to deploy and retract said grasping member (17).

5. A grasping device (1) according to claim 4, wherein said actuation section (7) includes a connecting block (32) affixed to said elongate control member (9) and is disposed within a longitudinal slot (4) of said handle (5).

6. A grasping device (1) according to any preceding claim, wherein said control member includes an outer rigid tube (21) and an inner flexible tube (20), the rigid tube extending through the control assembly and into the outer sheath and moveable therethrough.

7. A grasping device (1) according to any preceding claim, wherein said wire loops (50, 52, 54, 56, 70) are formed from a shape memory or superelastic metal or alloy, optionally wherein said wire loops (50, 52, 54, 56, 70) are formed of Nitinol.

8. A grasping device (1) according to any preceding claim, wherein the wire loops (50, 52, 54, 56, 70) have proximal end portions that are joined to said elongate control member (9) at an affixation point and said loops initially extend along said elongate control member even when said wire loops emerge from said distal end of said outer sheath (3) and self-deploy transversely of said grasping device (17, 71).

9. A grasping device (1) according to claim 8, wherein the affixation point comprises a spiral (63) of the wire from the wire loops (50, 52, 54, 56, 70) wound around the elongate control member (9).

10. A grasping device (1) according to any preceding claim, wherein said distal tip (15) comprises an atraumatic section.

11. A grasping device (1) according to any preceding claim, wherein at least one of said wire loops (50, 52, 54, 56, 70) includes a radio-opaque material.

12. A grasping device (1) according to claim 11, wherein the radio-opaque material comprises a coil (74) of platinum wire around and extending along the curved outer section of at least one of said loops.

13. A grasping device (1) according to any preceding claim, including a port fitting (40) on the control assembly between the elongate control member and the outer sheath operable to allow flushing with fluid to eliminate air.

14. A grasping device (1) according to claim 13, comprising a haemostatic seal (36, 37, 38) between the control assembly and the control member.

## Patentansprüche

1. Medizinische Greifvorrichtung (1), die Folgendes aufweist:
ein längliches Steuerglied (9), das mit einer distalen Spitze (15) und einem proximalen Endabschnitt versehen ist, wobei das längliche Steuerglied proximal zu der distalen Spitze ein Greifglied (17) aufweist,
eine äußere Hülle (3) mit einem Durchgang, in dem das längliche Steuerglied gleitend gehalten ist,
und eine Steueranordnung (2), die an einem proximalen Ende der äußeren Hülle und dem proximalen Endabschnitt des länglichen Steuerglieds angeordnet und dahingehend betreibbar ist, das Greifglied aus einem distalen Ende der äußeren Hülle und in dieses zu drängen,
wobei das Greifglied (17, 71) vier vorgeformte Drahtschlaufen (50, 52, 54, 56, 70) umfasst, die ungefähr gleichmäßig winkelmäßig um eine Längsachse des länglichen Steuerglieds (9) beabstandet sind und sich vom distalen Ende der äußeren Hülle selbst ausbringen, wobei jeweilige Enden jeder Drahtschlaufe an dem länglichen Steuerglied befestigt sind, wobei sich die Drahtschlaufen im ausgebrachten Zustand in verschiedenen Richtungen erstrecken und wobei sich benachbarte Drahtschlaufen gegenseitig überlappen,
**dadurch gekennzeichnet, dass** jede der Drahtschlaufen im vollständig ausgebrachten Zustand im Wesentlichen halbkreisförmig ist und jede Drahtschlaufe die benachbarte Drahtschlaufe zu beiden Seiten um ungefähr ein Viertel einer Gesamtkreisfläche überlappt, um ein allgemein rundes Greifglied bereitzustellen.

2. Greifvorrichtung (1) nach Anspruch 1, wobei das längliche Steuerglied eine flexible Kanüle (9) ist, die ein sich dort hindurch erstreckendes Lumen bereitstellt, in dem eine Drahtführung (19) gleitend aufnehmbar ist.

3. Greifvorrichtung (1) nach Anspruch 1 oder 2, wobei die äußere Hülle (3) flexibel und knickfest ist und eine schlüpfrige Außen- und/oder Innenfläche hat.

4. Greifvorrichtung (1) nach Anspruch 1, 2 oder 3, wobei die Steueranordnung ein ergreifbares Betätigungselement (7) aufweist, das entlang einem Griff (5) hin- und herbewegbar ist, um das längliche Steuerglied (9) bezüglich der äußeren Hülse (3) zu betätigen, um das Greifglied (17) auszubringen und zurückzuziehen.

5. Greifvorrichtung (1) nach Anspruch 4, wobei der Betätigungsabschnitt (7) einen an dem länglichen Steuerglied (9) befestigten Verbindungsblock (32) aufweist und in einem Längsschlitz (4) des Griffs (5) angeordnet ist.

6. Greifvorrichtung (1) nach einem der vorhergehenden Ansprüche, wobei das Steuerglied eine starre äußere Röhre (21) und eine flexible innere Röhre (20) aufweist, wobei sich die starre Röhre durch die Steueranordnung und in die äußere Hülle erstreckt und dort hindurch bewegbar ist.

7. Greifvorrichtung (1) nach einem der vorhergehenden Ansprüche, wobei die Drahtschlaufen (50, 52, 54, 56, 70) aus einem Formgedächtnismetall oder einer Formgedächtnislegierung oder einem superelastischen Metall oder einer superelastischen Legierung gebildet sind, wahlweise wobei die Drahtschlaufen (50, 52, 54, 56, 70) aus Nitinol gebildet sind.

8. Greifvorrichtung (1) nach einem der vorhergehenden Ansprüche, wobei die Drahtschlaufen (50, 52, 54, 56, 70) proximale Endabschnitte haben, die an einer Befestigungsstelle mit dem länglichen Steuerglied (9) verbunden sind, und sich die Schlaufen anfangs entlang dem länglichen Steuerglied erstrecken, selbst wenn die Drahtschlaufen aus dem distalen Ende der äußeren Hülle (3) austreten und sich quer zu der Greifvorrichtung (17, 71) selbst ausbringen.

9. Greifvorrichtung (1) nach Anspruch 8, wobei die Befestigungsstelle eine Spirale (63) des Drahts von den Drahtschlaufen (50, 52, 54, 56, 70) umfasst, der um das längliche Steuerglied (9) gewickelt ist.

10. Greifvorrichtung (1) nach einem der vorhergehenden Ansprüche, wobei die distale Spitze (15) einen atraumatischen Abschnitt umfasst.

11. Greifvorrichtung (1) nach einem der vorhergehenden Ansprüche, wobei mindestens eine der Drahtschlaufen (50, 52, 54, 56, 70) ein röntgendichtes Material aufweist.

12. Greifvorrichtung (1) nach Anspruch 11, wobei das röntgendichte Material eine Spirale (74) aus Platindraht umfasst, die um den gebogenen äußeren Abschnitt mindestens einer der Schlaufen verläuft und sich daran entlang erstreckt.

13. Greifvorrichtung (1) nach einem der vorhergehenden Ansprüche, einschließlich eines Port-Stutzens (40) an der Steueranordnung zwischen dem länglichen Steuerglied und der äußeren Hülle, der dahingehend betreibbar ist, zur Eliminierung von Luft Spülen mit Fluid zu gestatten.

14. Greifvorrichtung (1) nach Anspruch 13, umfassend eine hämostatische Dichtung (36, 37, 38) zwischen der Steueranordnung und dem Steuerglied.

## Revendications

1. Dispositif de préhension médical (1) comprenant :
un organe de commande allongé (9) comportant un embout distal (15) et une partie d'extrémité proximale, ledit organe de commande allongé comprenant un organe de préhension (17) en position proximale vis-à-vis dudit embout distal ;
une gaine extérieure (3) comportant un passage à l'intérieur duquel est supporté à coulissement l'organe de commande allongé ;
et un ensemble de commande (2) disposé au niveau d'une extrémité proximale de ladite gaine extérieure et de ladite partie d'extrémité proximale dudit organe de commande allongé et pouvant être mis en oeuvre afin de solliciter ledit organe de préhension de façon à ce qu'il sorte par une extrémité distale de ladite gaine extérieure et rentre dans celle-ci ;
ledit organe de préhension (17, 71) comprenant quatre boucles de fil préformées (50, 52, 54, 56, 70) espacées de manière approximativement équidistante angulairement autour d'un axe longitudinal dudit organe de commande allongé (9), qui se déploient automatiquement à partir de ladite extrémité distale de ladite gaine extérieure, des extrémités respectives de chaque boucle de fil étant fixées à l'organe de commande allongé ; lesdites boucles de fil, une fois déployées, s'étendant dans des directions différentes et des boucles de fil adjacentes se chevauchant l'une l'autre ;
**caractérisé en ce que** chacune desdites boucles de fil est essentiellement semi-circulaire lorsqu'elle est totalement déployée et chaque boucle de fil chevauche la boucle voisine de chaque côté d'approximativement un quart d'une aire de cercle totale de façon à former un organe de préhension globalement rond.

2. Dispositif de préhension (1) selon la revendication 1, dans lequel ledit organe de commande allongé est une canule souple (9) formant une lumière s'étendant à travers elle, dans laquelle un fil-guide (19) peut être reçu à coulissement.

3. Dispositif de préhension (1) selon la revendication 1 ou 2, dans lequel ladite gaine extérieure (3) est souple et résistante à la déformation et comporte une surface extérieure et/ou intérieure lubrifiée.

4. Dispositif de préhension (1) selon la revendication 1, 2 ou 3, dans lequel ledit ensemble de commande comprend un élément d'actionnement préhensible (7) pouvant être déplacé en avant et en arrière le long d'une poignée (5) afin d'actionner ledit organe de commande allongé (9) par rapport à ladite gaine extérieure (3) de façon à déployer et rétracter ledit organe de préhension (17).

5. Dispositif de préhension (1) selon la revendication 4, dans lequel ladite section d'actionnement (7) comprend un bloc de raccordement (32) fixé audit organe de commande allongé (9) et est disposée à l'intérieur d'une fente longitudinale (4) de ladite poignée (5).

6. Dispositif de préhension (1) selon l'une quelconque des revendications précédentes, dans lequel ledit organe de commande comprend un tube rigide extérieur (21) et un tube souple intérieur (20), le tube rigide s'étendant à travers l'ensemble de commande et dans la gaine extérieure et pouvant être déplacé à travers ceux-ci.

7. Dispositif de préhension (1) selon l'une quelconque des revendications précédentes, dans lequel lesdites boucles de fil (50, 52, 54, 56, 70) sont constituées d'un métal ou d'un alliage à mémoire de forme ou superélastique, éventuellement dans lequel lesdites boucles de fil (50, 52, 54, 56, 70) sont constituées de Nitinol.

8. Dispositif de préhension (1) selon l'une quelconque des revendications précédentes, dans lequel les boucles de fil (50, 52, 54, 56, 70) comportent des parties d'extrémité proximales qui sont raccordées audit organe de commande allongé (9) au niveau d'un point de fixation et lesdites boucles s'étendent initialement le long dudit organe de commande allongé même lorsque lesdites boucles de fil émergent de ladite extrémité distale de ladite gaine extérieure (3) et se déploient automatiquement transversalement vis-à-vis dudit dispositif de préhension (17, 71).

9. Dispositif de préhension (1) selon la revendication 8, dans lequel le point de fixation comprend une spirale (63) du fil appartenant aux boucles de fil (50, 52, 54, 56, 70) enroulée autour de l'organe de commande allongé (9).

10. Dispositif de préhension (1) selon l'une quelconque des revendications précédentes, dans lequel ledit embout distal (15) comprend une section atraumatique.

11. Dispositif de préhension (1) selon l'une quelconque des revendications précédentes, dans lequel au moins une desdites boucles de fil (50, 52, 54, 56, 70) comprend un matériau radio-opaque.

12. Dispositif de préhension (1) selon la revendication 11, dans lequel le matériau radio-opaque comprend un enroulement (74) de fil de platine autour et s'étendant le long de la section extérieure incurvée d'au moins une desdites boucles.

13. Dispositif de préhension (1) selon l'une quelconque des revendications précédentes, comprenant une pièce formant orifice (40) sur l'ensemble de commande entre l'organe de commande allongé et la gaine extérieure, pouvant être mise en oeuvre de façon à permettre le rinçage avec un fluide afin d'éliminer l'air.

14. Dispositif de préhension (1) selon la revendication 13, comprenant un joint hémostatique (36, 37, 38) entre l'ensemble de commande et l'organe de commande.
